# EUROPEAN PATENT SPECIFICATION

(11) **EP 1 777 307 B1**
(45) Date of publication and mention of the grant of the patent: **01.02.2012**
(21) Application number: 05770446.2
(22) Date of filing: 10.08.2005
(51) Int. Cl.: C22C 9/00, B22D 21/00, C22C 9/02

(54) **Sn-CONTAINING COPPER ALLOY AND METHOD FOR PRODUCTION THEREOF**
SN-HALTIGE KUPFERLEGIERUNG UND HERSTELLUNGSVERFAHREN DAFÜR
ALLIAGE DE CUIVRE CONTENANT DU Sn ET MÉTHODE DE PRODUCTION RELATIVE

(30) Priority: 10.08.2004 JP 2004233952
(43) Date of publication of application: 25.04.2007
(73) Proprietor: Mitsubishi Shindoh Co., Ltd., Shinagawa-ku Tokyo (JP)
(72) Inventor: OISHI, Keiichiro, 581-0032 (JP)
(74) Representative: HOFFMANN EITLE
(86) International application number: PCT/JP2005/014699
(87) International publication number: WO 2006/016631

(56) References cited:
- WO-A-99/67433
- WO-A1-94/10352
- JP-A- 3 291 344
- JP-A- 8 127 830
- JP-A- 11 001 736
- JP-A- 50 078 519
- JP-A- 52 107 227
- JP-A- 61 000 542
- JP-A- 62 274 036
- JP-A- 2001 247 923
- JP-A- 2004 143 541
- US-A- 4 822 560

## Description

### Technical Field

The present invention relates to a Sn-containing copper alloy, the castability, hot cold workability or the like of which are improved by refining the grains, and to a method of manufacturing the same.

### Background Art

Generally, a phosphor bronze or the like defined in C5111, C5102, C5191 and C5212 of JIS H3110 or JIS H3270; CAC502A and CAC502B of JIS H5120; C4250 and C4430 of JIS H3100 are well known as a Sn-containing copper alloy. However, such copper alloy has inferior castability, hot cold workability or the like, and thus can be used only in limited fields. The above disadvantages are due to Sn, a low melting point metal, contained in the copper alloys, particularly, to the macro-structural segregation of Sn.

As an effective method of preventing such macro-structural segregation of Sn, it can be considered to refine the grains of the copper alloys.

Generally, the grains of the copper alloys are refined as follows: (A) the grains are refined during the melt-solidification of the copper alloys, or (B) the grains are refined by deforming, such as rolling or the like, or heating the melt-solidified copper alloys (ingot such as slurry or the like, casting product such as die casting or the like, solid metal casting or the like), in which stored energy such as distortion energy or the like acts as a driving force. In any case, Zr is known as an effective element for the grain refinement.

However, in the case of (A), the action of Zr to refine the grains during the melt-solidification significantly depends on the other elements and the amount thereof, therefore, it is hard to achieve a desired level of grain refinement. Consequently, the method in the case of (B) is widely used, that is, melt-solidified ingot, casting product or the like is provided with heat treatment and then distorted in order to refine the grains.

For example, JP-B-38-20467 discloses that the grains are refined further as the content of Zr increases on the basis of an investigation, in which copper alloys containing Zr, P and Ni are solution-treated and then cold-worked at the processing rate of 75%, showing the mean grain sizes 280 µm (no Zr contained), 170 µm (0.05 mass% of Zr contained), 50 µm (0.13 mass% of Zr contained), 29 µm (0.22 mass% of Zr contained) and 6 µm (0.89 mass% of Zr contained). In addition, JP-B-38-20467 proposes that the content of Zr be in the range of 0.05 to 0.3 mass% in order to avoid bad influence from the excessive content of Zr.

Furthermore, JP-A-2004-233952 discloses that, if copper alloys containing 0.15 to 0.5 mass% of Zr are provided with solution treatment and deforming process for distorting the alloys after casting, the mean grain sizes are reduced to be about 20 µm or less.

However, if the copper alloys are solution-treated and deformed after casting to refine the grains like the method in the case of (B), the manufacturing cost will rise. In addition, there can be cases where the deforming process for distorting the copper alloys is hardly performed due to the shapes of the copper alloy casting products. Therefore, it is preferable that the grains be refined during the melt-solidification of copper alloy by the method in the case of (A). However, in the case of (A), since the action of Zr significantly depends on the other elements and the content thereof as described above, the grains are not necessarily refined as much as the content of Zr increases. In addition, since Zr has an extremely high affinity to oxygen, Zr is highly likely to be an oxide when melted and added in the air, thereby reducing the yield of Zr. Consequently, even when a casting product contains a slight amount of Zr, a great amount of Zr needs to be charged during pouring. Meanwhile, if too many oxides are generated during melting, it is highly likely that the oxides enter the molten alloy during the pouring, thereby easily generating casting defects in the casting products. Even though the melting or casting can be carried out under vacuum or inert gas atmosphere in order to prevent the oxide from being generated, the manufacturing cost would significantly rise in this case. In addition, since Zr is an expensive element, it is preferable from an economic viewpoint that the adding amount of Zr be as low as possible.

JP-A-8-127830 is concerned with the production of copper alloys for wire conductors. The ingots of a copper alloy are stated as having a composition consisting of, by weight, 0.8-2.0% Sn, 0.03-0.1%P, 0.008-0.02 Zr, and the balance Cu with inevitable impurities.

As a result, it is highly demanded to develop a method to reduce the mean grain size during the melt-solidification while the content of Zr remains as low as possible.

### Disclosure of the Invention

The invention has been finalized in terms of the drawbacks inherent to the related arts, and it is an advantage of an aspect of the invention to provide a Sn-containing copper alloy, in which grains are refined and the macro-structural segregation of Sn seldom occurs so as to exclude the inherent drawbacks such as the deterioration of castabiliy, hot · cold workability or the like with no damages on the effect of Sn (the improvement of corrosion resistance, strength or the like), and a method of manufacturing the same.

In order to achieve the advantage, the invention proposes, particularly, the following Sn-containing copper alloy and method of manufacturing the same.

That is, according to a first aspect of the invention, the invention proposes a Sn-containing copper alloy (hereinafter referred to as 'first copper alloy') containing Sn: 0.01 to 16 mass% (preferably 0.3 to 15 mass%, more preferably 0.5 to 13 mass%, further more preferably 0.7 to 11 mass%, and most preferably 0.8 to 3.5 mass%); Zr: 0.001 to 0.049 mass% (preferably 0.003 to 0.039 mass%, more preferably 0.0055 to 0.029 mass%, further more preferably 0.0075 to 0.024 mass%, and most preferably 0.0085 to 0.019 mass%); P: 0.01 to 0.25 mass% (preferably 0.02 to 0.18 mass%, more preferably 0.025 to 0.14 mass%, further more preferably 0.03 to 0.12 mass%, and most preferably 0.035 to 0.11 mass%); Zn: 0.01 to 38 mass% (preferably 10 to 37 mass%, more preferably 15 to 36 mass%, and most preferably 20 to 34 mass%); optionally one or more elements selected from Mn: 0.05 to 4 mass%, Al: 0.01 to 3 mass%, Si: 0.01 to 1.9 mass%, Co: 0.005 to 0.1 mass%, As: 0.02 to 0.2 mass%, Sb: 0.02 to 0.2 mass% and Mg: 0.001 to 0.2 mass%; optionally Fe and/or Ni as inevitable impurities, wherein when either of Fe or Ni is included, the content thereof is limited to be 0.25 mass% or less, and when both of Fe and Ni are included, the total content thereof is limited to be 0.3 mass% or less; and Cu: a remainder, satisfying the conditions f0 to f4 as defined in the appending claim 1. In the first copper alloy, α-phase and one or both of γ-phase and δ-phase are included and the total content of the α-phase and one or both of γ-phase and δ-phase reaches 90% or more by area ratio, and a mean grain size in a macrostructure after meltsolidification is 300 µm or less. That is, the first copper alloy satisfies the following conditions (1) to (7). The first copper alloy is preferable to further satisfy the following conditions (8) to (13) in addition to the conditions (1) to (7).

According to a second aspect of the invention, the invention proposes a Sn-containing copper alloy (hereinafter referred to as 'second copper alloy') further containing one or more elements selected from Mn, Al, Si and Co in addition to the components of the first copper alloy, that is, additionally containing one or more elements selected from Mn: 0.05 to 4 mass% (preferably 0.03 to 3.5 mass% and more preferably 0.05 to 3 mass%), Al: 0.01 to 3 mass% (preferably 0.05 to 2.5 mass% and more preferably 0.1 to 2.3 mass%), Si: 0.01 to 1.9 mass% (preferably 0.02 to 1.5 mass% and more (preferably 0.05 to 1.2 mass%) and Co: 0.005 to 0.1 mass% (preferably 0.01 to 0.05 mass% and more preferably 0.01 to 0.03 mass%).

According to a third aspect of the invention, the invention proposes a Sn-containing copper alloy (hereinafter referred to as 'third copper alloy') further containing one or more elements selected from As, Sb and Mg in addition to the components of the first copper alloy, that is, additionally containing one or more elements selected from As: 0.02 to 0.2 mass% (preferably 0.03 to 0.12 mass%), and Sb: 0.02 to 0.2 mass% (preferably 0.03 to 0.12 mass%) and Mg: 0.001 to 0.2 mass% (preferably 0.002 to 0.15 mass% and more preferably 0.005 to 0.1 mass%).

According to a fourth aspect of the invention, the invention proposes a Sn-containing copper alloy (hereinafter referred to as 'fourth copper alloy') further containing one or more elements selected from Mn, Al, Si and Co; and one or more elements selected from As, Sb and Mg in addition to the components of the first copper alloy, that is, further containing one or more elements selected from As: 0.02 to 0.2 mass% (preferably 0.03 to 0.12 mass%), and Sb: 0.02 to 0.2 mass% (preferably 0.03 to 0.12 mass%) and Mg: 0.001 to 0.2 mass% (preferably 0.002 to 0.15 mass%, more preferably 0.005 to 0.1 mass%); one or more elements selected from Mn: 0.05 to 4 mass% (preferably 0.03 to 3.5 mass% and more preferably 0.05 to 3 mass%), Al: 0.01 to 3 mass% (preferably 0.05 to 2.5 mass% and more preferably 0.1 to 2.3 mass%), Si: 0.01 to 1.9 mass% (preferably 0.02 to 1.5 mass% and more preferably 0.05 to 1.2 mass%) and Co: 0.005 to 0.1 mass% (preferably 0.01 to 0.05 mass% and more preferably 0.01 to 0.03 mass%).

The second, third and fourth copper alloys each preferably further satisfy the following conditions (8) to (13) in addition to the conditions (1) to (7).

Meanwhile, in the following description, the content of element 'a' is denoted by [a] and expressed in a unit of 'mass%'. For example, the content of Cu is expressed as [Cu] mass%.
(1) f0 = [Cu] - 0.5 [Sn] - 3[P] - 0.5([As] + [Sb]) + [Mg] + [Mn] - 1.8 [Al] - 3.5[Si] = 61 to 97 (preferably 62.5 to 90, more preferably 63.5 to 88 and most preferably 65 to 75). Meanwhile, in f0, an element 'a', not contained in the copper alloy, is denoted by [a] = 0. For example, the first copper alloy satisfies f0 = [Cu] - 0.5[Sn] - 3[P].
(2) f1 = [P] / [Zr] = 0.5 to 100 (preferably 0.8 to 25, more preferably 1.1 to 16, and most preferably 1.5 to 12).
(3) f2 = ([Zn] + 3[Sn]) / [Zr] = 30 to 15000 (preferably 300 to 7000, more preferably 650 to 5000 and most preferably 1000 to 3000). Meanwhile, in f2, an element 'a', not contained in the copper alloy, is denoted by [a] = 0. For example, for the first to fourth copper alloys not containing Zn, f2 = 3[Sn] / [Zr].
(4) f3 = ([Zn] + 3[Sn]) / [P] = 3 to 2500 (preferably 60 to 1600, more preferably 120 to 1200, and most preferably 200 to 800). Meanwhile, in f3, an element 'a', not contained in the copper alloy, is denoted by [a] = 0. For example, the first to four copper alloys containing no Zn satisfy f3 = 3 [Sn] / [P].
(5) α phase and, γ and/or δ-phases are contained, and the total content of α phase and, γ and/or δ-phases occupy 90% or more (preferably 98% or more, and more preferably 99% or more) of the total area of the copper alloy. Meanwhile, the content of each phase, that is, area ratio, is measured by image analysis, more specifically, by expressing the structure of the copper alloy, 200 times magnified by an optical microscope, in the binary system with an image processing software 'WinROOF' (manufactured by Tech-Jam Co., Ltd.). The content of each phase is an average value of the area ratios measured in three different fields.
(6) The mean grain size of the macrostructure after melt-solidification is 300 µm or less (preferably 200 µm or less, more preferably 100 µm or less and the mean grain size of the microstructure is 60 µm or less). Herein, the mean grain size of the macrostructure (or microstructure) after the melt-solidification represents the average value of the grain size of the macrostructure (or microstructure) when no deforming process or heat treatment is carried out on the copper alloy melt-solidified.by casting (including casting by various common casting methods such as permanent mold casting, sand casting, horizontal continuous casting, upward (upcast), semi-solid metal casting, semi-solid metal forging, melt-solidification forging or the like) or either welding or fusing. Meanwhile, in this description, 'casting' or 'casting product' represents a product, all or part of which is melted and solidified, for example, beginning from ingot, slab and billet for rolling or extruding, sand casting product, permanent mold casting product, low-pressure casting product, die casting, lost wax, semi-solid metal casting (for example, thixo-casting or rheo-casting) semi-solid metal forming product, squeeze, centrifugal casting, continuous casting product (for example, rod, hollow rod, irregular shape rod, irregular shape hollow rod, coil, wire or the like manufactured by horizontal continuous casting, upward or upcast) and casting product manufactured by melt forging (direct forging), metallizing, build-up spraying, lining and overlay. In addition, welding can be considered a sort of casting in a broad sense, since, in the welding, part of mother materials are melted and solidified so as to join the mother materials.
(7) f4 = [Zn] + 3[Sn] = 10 to 48 (preferably 15 to 45, more preferably 20 to 40, and most preferably 25 to 38).
(8) A primary crystal during the melt-solidification is α-phase.
(9) Peritectic reaction occurs during the meltsolidification.
(10) During the melt-solidification, dendrite network is divided in the crystal structure, and two-dimensional shape of the crystal grains is circular, substantially circular, oval, cross-like, acicular or polygonal.
(11) α-phase in the matrix is divided minutely, and δ-phase, γ-phase or high Sn-concentration part generated by segregation is distributed uniformly in the matrix.
(12) In a semi-solid metal state, in which the ratio of solid phase reaches 30 to 80%, at least dendrite network is divided in the crystal structure, and the two-dimensional shape of the solid phase is circular, substantially circular, oval, cross-like or polygonal.
(13) In a semi-solid metal state, in which the ratio of the solid phase reaches 60%, the mean grain size of the solid phase is 150 µm or less (preferably 100 µm or less, more preferably 50 µm or less, and most preferably 40 µm or less), and/or the mean maximum length of the solid phase is 200 µm or less (preferably 150 µm or less, more preferably 100 µm or less, and most preferably 80 µm or less).

In addition, in the first to fourth copper alloys, Cu is a main element of the copper alloys composing the casting products, and as the content of Cu increases, ① α-phase is easily obtained, ② the corrosion resistance (dezincification corrosion resistance and stress corrosion cracking resistance) improves, and ③ mechanical properties improve. However, since the excessive content of Cu impedes the grain refinement, the content of Cu composes the remainder. Furthermore, while depending on the proportion of Cu to Sn (and Zn), it is preferable that the content of Cu be in the range having the minimum value, at which the copper alloy can secure more stable corrosion resistance and erosion resistance, and the maximum value, at which the copper alloy can secure more stable strength and wear resistance. Still furthermore, it is required to consider the relationship with the other elements in order to refine the grains. Consequently, the content of Cu is required to compose the remainder and to satisfy the condition (1). That is, the contents of Cu and the other elements have to meet f0 = [Cu] - 0.5[Sn] - 3[P] - 0.5 ([As] + [Sb]) + [Mg] + [Mn] - 1.8[Al] - 3.5[Si] = 61 to 97, preferably 62.5 to 90, more preferably 63.5 to 88, and most preferably 65 to 75. Meanwhile, the minimum value of f0 is also related with whether the primary crystal is α-phase, and the maximum value of f0 is also related with the peritectic reaction.

In the first to fourth copper alloys, Sn is contained mainly to improve the corrosion resistance. If 0.01 mass% or more of Sn is added, Sn can improve the corrosion resistance, erosion resistance, wear resistance and strength. However, the above effect saturates when the adding content of Sn reaches 16 mass%, and the ductility and castability deteriorate inversely if more than 16 mass% of Sn is added even in the presence of Zr and P, thereby causing casting defects such as crack, shrinkage cavity, porosity or the like. In addition, Sn widens the composition range capable of generating peritectic reaction (an effective method to refine the grains during the melt-solidification), and, practically, peritectic reaction can occur throughout the wide concentration range of Cu as the content of Sn increases. Considering the above facts, the content of Sn is defined as preferably 0.3 mass% or more, more preferably 0.5 mass% or more, further more preferably 0.7 mass% or more, and most preferably 0.8 mass% or more. On the other hand, while depending on the proportion of Sn to Cu and Zn, if the content of Sn exceeds 16 mass%, δ-phase (and γ-phase), a hard phase with the Sn-concentration higher than that of mother phase (α-phase), is generated excessively (exceeding 20% by area ratio), thereby causing selective corrosion of the phase and, consequently, impairing the corrosion resistance. Furthermore, while depending on the proportion of Sn to Cu (Sn to Cu and Zn in the cases of the fifth to eighth copper alloys), if the concentration of Sn is too high, Sn is segregated too much, and the solidification temperature range is widened as the adding amount of Sn increases, thereby impairing the castability. Considering the above facts, the content of Sn needs to be defined as 0.01 to 16 mass%, preferably 0.3 to 15 mass%, more preferably 0.5 to 13 mass%, further more preferably 0.7 to 11 mass%, and most preferably 0.8 to 3.5 mass%. If the content of Sn is in the above range, the content of δ-phase (and γ-phase) can remain at a proper level (20% or less by area ratio).

In the first to fourth copper alloys, Zr and P are added together to refine the grains of the copper alloy, particularly, during the melt-solidification. That is, even though either Zr or P alone can refine the grains slightly like the other additives, if added together, Zr and P can refine the grains effectively.

With respect to Zr, even though the grains can be refined when 0.001 mass% or more of Zr is contained, the grains can be refined remarkably at the content of 0.003 mass% or more, more remarkably at the content of 0.0055 mass%, further more remarkably at the content of 0.0075 mass% or more, and most remarkably at the content of 0.0085 mass% or more. With respect to P, even though the grains can be refined when 0.01 mass% or more of P is contained, the grains can be refined remarkably at the content of 0.02 mass% or more, more remarkably at the content of 0.025 mass%, further more remarkably at the content of 0.03 mass% or more, and most remarkably at the content of 0.035 mass% or more.

On the other hand, when the content of Zr reaches 0.049 mass% and the content of P reaches 0.25 mass%, the action of Zr and P to refine the grains saturates regardless of the type and content of the other elements, and, inversely, the action of Zr and P to refine the grains deteriorates. Therefore, the content of Zr needs to be 0.049 mass% or less and the content of P needs to be 0.25 mass% or less in order to refined the grains effectively. If the adding amounts of Zr and P are in the above narrow ranges, the features of the alloy induced by the other elements are not impaired, and further high Sn-concentration parts caused by segregation can be distributed uniformly in the matrix by the grain refinement, instead of being concentrated at certain areas. Consequently, casting crack can be prevented, and a robust casting with less porosity, shrinkage cavity, blowhole and micro-porosity can be obtained. In addition, the workability for cold drawing or the like, which is performed after casting, can be improved, and thus the features of the alloy can be further improved.

Meanwhile, since Zr has a strong affinity to oxygen, Zr is highly likely to be oxidized or sulfurated when Zr is melted in the air or a scrap material is used as raw material. In addition, if Zr is added excessively, the viscosity of the molten alloy increases, therefore, zirconium oxide or sulfide can enter the molten alloy so as to generate casting defects during casting, which leads to the generation of blowhole or micro-porosity. Even though it can be considered to melt the raw material and then cast the molten alloy under vacuum or complete inert gas atmosphere to avoid the above problems, this method cannot be used widely and raises the cost considerably when Zr is added to copper alloy only for grain refinement. Considering the above facts, the adding amount of Zr, not in the form of oxide or sulfide, is preferably 0.039 mass% or less, more preferably 0.029 mass% or less, further more preferably 0.024 mass% or less, and most preferably 0.019 mass% or less. In addition, if the amount of Zr is in the above range, less zirconium oxide or sulfide is generated even when the casing product is melted in the air for recycling, and thus the robust first to eighth copper alloys composed of fine grains can be obtained again. Furthermore, the grains are not refined further even when the adding amount of Zr exceeds 0.029 mass%, and the grain refinement effect almost saturates even when the adding amount of Zr exceeds 0.039 mass%.

From the above facts, only a small amount of Zr needs to be added industrially, therefore, the adding amount of Zr needs to be in the range of 0.001 to 0.049 mass%, preferably 0.003 to 0.039 mass%, more preferably 0.0055 to 0.029 mass%, further more preferably 0.0075 to 0.024 mass%, and most preferably 0.0085 to 0.019 mass%.

In addition, even though P is added with Zr to refine the grains as described above, P also influences on the corrosion resistance, castability or the like. Therefore, considering the influence of the minimum amount of P on the corrosion resistance, castability or the like and the influence of the maximum amount of P on the ductility of the like, the adding amount of P needs to be in the range of 0.01 to 0.25 mass%, preferably 0.02 to 0.18 mass%, more preferably 0.025 to 0.14 mass%, further more preferably 0.03 to 0.12 mass%, and most preferably 0.035 to 0.11 mass%.

Furthermore, in order for co-added Zr and P to refine the grains, the contents of Zr and P have to satisfy the condition (2) as well as be in the above ranges individually. The grains are refined when the nucleation rate of α-phase, the primary crystal crystallized from the molten alloy, is much faster than the growth rate of dendrite crystal. However, in order to make the nucleation rate of α-phase much faster than the growth rate of dendrite crystal, not only are the adding amounts of Zr and P determined individually, but the ratio of the adding amounts of P and Zr (f1 = [P] / [Zr]) also have to be considered. The function or interaction of Zr and P can facilitate the crystal growth of the primary α-phase remarkably when the contents of Zr and P are determined individually to satisfy the proper ratio of the adding amounts in the proper ranges, and then the nucleation rate of α-phase becomes much faster than the growth rate of the dendrite crystal. If the contents of Zr and P are in the proper ranges, and the proportion thereof ([P] / [Zr]) is stoichiometric, an intermetallic compound of Zr and P (for example, ZrP or ZrP₁₋ₓ) can be generated in α-phase crystal by adding a minute amount of Zr, for example, several tens ppm, therefore, the nucleation rate of α-phase becomes faster when f1, the value of [P] / [Zr], is in the range of 0.5 to 100, further faster when f1 is in the range of 0.8 to 25, still further faster when f1 is in the range of 1.1 to 16, fastest when f1 is in the range of 1.5 to 12. That is, the ratio f1 of the adding amounts of Zr and P is an important factor for the grain refinement, and, when f1 is in the above range, the nucleation rate is much faster than the crystal growth rate during the melt-solidification. Still furthermore, for the grain refinement, the ratios of the adding amounts of Zr, P to Sn (Zr, P to Sn and Zn when Zn is contained) (f2 = ([Zn] + 3[Sn]) / [Zr], f3 = ([Zn] + 3[Sn]) / [P]) and f4 = [Zn] + 3[Sn] are considerably important, thus need to be considered, and have to satisfy the conditions (3), (4) and (7).

Still furthermore, as the melt-solidification proceeds and the ratio of solid phase increases, the crystal growth occurs frequently and part of the grains begin to combine one another, therefore, the size of α-phase crystal increases. In this case, if peritectic reaction occurs during the solidification of the molten alloy, the molten alloy, not solidified yet, is reacted with the solid α-phase, thereby generating β-phase while the amount of solid α-phase decreases. As a result, α-phase is folded with β-phase, therefore, the size of α-phase grain decreases and the shape of α-phase grain becomes an oval with smoothed edges. If the solid phase is fine and oval, gas can be removed easily, and the copper alloy has a resistance to crack accompanied by solidification shrinkage during solidification, thereby the copper alloy is shrunk smoothly, and various features such as strength, corrosion resistance or the like at the room temperature are improved. It is needless to say that, if the solid phase is fine and oval, the flowability is good, and the copper alloy is most preferable to the semi-solid metal solidifying method. In addition, if fine and oval solid phase and molten alloy remain at the final stage of the solidification, sufficient amount of the solid phase and the molten alloy can fill every corner even in a complex-shaped mold, thereby manufacturing excellently shaped casting products. That is, even a near net shape can be molded. Meanwhile, in practice, that is, not in the equilibrium state, peritectic reaction occurs in a wider composition range than the composition range at the equilibrium state. Herein, the equations f0 and f4 play an important role, that is, the maximum of f0 (the minimum of f4) is related mainly with a criterion for the grain size after the melt-solidification and the occurrence of the peritectic reaction. The minimum of f0 (the maximum of f4) is related mainly with the crystal size after the melt-solidification and the boundary value that determines whether the primary crystal is α-phase. As f0 and f4 satisfy the above preferable range, more preferable range and most preferable range, the amount of the primary α-phase increases, and peritectic reaction, which occurs at a nonequilibrium reaction, occurs actively, therefore, the grains obtained at the room temperature becomes smaller.

The above melt-solidification phenomena depend on the cooling rate. That is, in the case of rapid cooling that cools an alloy at a rate of 10⁵ °C/sec or more, there is no enough time for nucleation, therefore, the grains are likely not to be refined. On the other hand, in the case of slow cooling that cool an alloy at a rate of 10⁻³ °C/sec or less, the grain growth is facilitated, thereby the grains are likely not to be refined. In addition, since the state approaches the equilibrium state, the composition range that generates peritectic reaction becomes narrower. The cooling rate at the melt-solidification stage is more preferably in the range of 10⁻² to 10⁴ °C/sec, and most preferably in the range of 10⁻¹ to 10³ °C/sec. Even in the above cooling rate range, as the cooling rate approaches the maximum, the composition range, in which the grains are refined, is widened, therefore, the grains are further refined.

Meanwhile, in the first to fourth copper alloys, even though Sn alone refines the grains slightly, Sn can refine the grains remarkably in the presence of Zr and P. Sn improves the mechanical properties (particularly, tensile strength, proof stress, fatigue strength and impact strength), corrosion resistance and wear resistance. In addition, Sn divides dendrite arms and widens the composition range of Cu or Zn, in which peritectic reaction occurs, so as to generate more effective peritectic reaction. Furthermore, Sn reduces the stacking fault energy of the alloy. As a result, even though Sn granulates and refines the grains effectively, this effect can be obtained more effectively when Sn exists with Zr and P. In addition, δ and γ-phases (mainly δ-phase), generated by the addition of Sn, suppress the grain growth after the melt-solidification, thereby contributing to the grain refinement. Even though δ-phase (and γ-phase) is an area transformed from high Sn-concentration parts, the high Sn-concentration parts are distributed uniformly and minutely at the melt-solidification stage, therefore, phase (and γ-phase) is also distributed minutely so as to suppress the growth of α-phase crystal grains in the high temperature region after the solidification. Furthermore, since δ-phase (and γ-phase) is distributed minutely, the corrosion resistance and wear resistance are good. Therefore, in order to make the co-added Zr and P refine the grains effectively, it is necessary that the contents of Zr and P be determined in consideration of the relationship between the contents of Zr and P and the relationship among the above contents and the content of Sn (and Zn) and that the contents of Zr and P satisfy the conditions (3) and (4) in addition to the condition (2). That is, in making the co-added Zr and P refine the grains effectively, in addition to the above relationship between the contents of Zn and P, f2 (= ([Zn] + 3[Sn]) / [Zr]), the content ratio of Zr to Sn and Zn, and f3 (= ([Zn] + 3[Sn]) / [P]), the content ratio of P to Sn and Zn, are also important factors, therefore, it is necessary that f2 be in the range of 30 to 15000 and f3 be in the range of 3 to 2500 while f1 is in the range of 0.5 to 100. The degree of the grain refinement arising from the co-addition of Zr and P becomes larger when f1 = 0.8 to 25, f2 = 300 to 7000, and f3 = 60 to 1600, further larger when f1 = 1.1 to 16, f2 = 650 to 5000, and f3 = 120 to 1200, and largest when f1 = 1.5 to 12, f2 = 1000 to 3000, and f3 = 200 to 800.

Similar to Sn, Zn that is contained in the first to fourth copper alloys generates peritectic reaction, which is a powerful method to refine the grains during the melt-solidification of the copper alloy, and reduces the stacking fault energy of the copper alloy so as to improve the flowability of the molten alloy and to facilitate the lowering of the melting point. In addition, Zn improves the corrosion resistance and mechanical features (tensile strength, proof stress, impact strength, wear resistance, fatigue resistance or the like). Furthermore, Zn facilitates the grain refinement at the melt-solidification and prevents Zr from being oxidized. However, if a great amount of Zn is added, the primary crystal becomes β-phase at the melt-solidification, therefore, it becomes hard to satisfy the conditions (8) to (11). Considering the above facts, the contents of Zn needs to be in the range of 0.01 to 38 mass%, preferably in the range of 10 to 37 mass%, more preferably in the range of 15 to 36 mass%, and most preferably in the range of 20 to 34 mass%.

In the third and fourth copper alloys, As, Sb and Mg are added mainly to improve the corrosion resistance. Even though the corrosion resistance can be improved when 0.02 mass% or more of Sb and/or As are added and 0.001 mass% or more of Mg is added, in order to improve the corrosion resistance remarkably, it is preferable that 0.03 mass% or more of Sb and/or As be added and 0.002 mass% or more of Mg be added. Furthermore, it is more preferable that 0.005 mass% or more of Mg be added. On the other hand, even when the adding amount of Sb or As exceeds 0.2 mass%, the corresponding effect cannot be obtained, and the ductility deteriorates inversely. In addition, the copper alloy becomes toxic. Considering the above facts, the adding amount of Sb or As needs to be 0.2 mass% or less and preferably 0.12 mass% or less. Meanwhile, even though it is common to use a scrap material (scrapped heat pipe) as a raw material for the copper alloy, and such scrap material contains S-component (sulfur-component), if the S-component is contained in the molten alloy, Zr, a grain-refining element, is likely to be sulfurated so as not to contribute to the grain refinement and further to impair the flowability, therefore, casting defects such as blowhole, crack or the like are highly likely to occur. Not only does Mg improve the corrosion resistance, but Mg also improves the flowability of the molten alloy when a scrap material containing the S-component is used for casting as a raw material of an alloy. In addition, Mg can remove the S-component in the form of MgS, which is less harmful than the S-component and not harmful to the corrosion resistance even when MgS remains in the alloy, and prevent the deterioration of the corrosion resistance caused by the S-component contained in the raw material. Furthermore, if the raw material contains the S-component, the S-component is highly likely to exist in the grain boundary so as to cause the intergranular corrosion. However, the intergranular corrosion can be effectively prevented by adding Mg. Still furthermore, even though it is likely that the concentration of S in the molten alloy becomes high and thus Zr is consumed by S, if 0.001 mass% or more of Mg is contained in the molten alloy before the charging of Zr, the S-component in the molten alloy is removed or fixed in the form of MgS, thereby preventing such problem. However, if Mg is added excessively, for example, more than 0.2 mass%, Mg is oxidized like Zr, and the viscosity of the molten alloy increases, thereby generating casting defects caused by the inclusion of the oxide or the like. Therefore, considering the above facts, the adding amount of Mg needs to be in the range of 0.001 to 0.2 mass%, preferably 0.002 to 0.15 mass%, and more preferably 0.005 to 0.1 mass%.

In the second and fourth copper alloys, Al, Mn, Si and Co are added mainly to improve the strength. Al, Mn and Sn induce the improvement of the flowability of the molten alloy, deoxidation, desulfuration, the improvement of the erosion resistance and the improvement of wear resistance under a strong current as well as the improvement of the strength. Particularly, Al and Si form robust Al - Sn and Si - Sn corrosion resistant coatings individually on the surface of a casting so as to improve the erosion resistance. In addition, Mn also forms the corrosion resistant coating between Sn and Mn. Furthermore, even though not as much as Mg, Al and Mn also remove the S-component in the molten alloy. Still furthermore, when a large amount of oxygen exists in the molten alloy, Zr is oxidized so as not to refine the grains, and, in this case, Al and Mn prevent Zr from being oxidized. Si deoxidizes and further reduces the stacking fault energy of the alloy if contained with Zr, P, Cu and Zn so as to refine the grains remarkably. Furthermore, Si improves the flowability of the molten alloy, prevents the molten alloy from being oxidized, and reduces the melting point, thereby improving the corrosion resistance, particularly, dezincification corrosion resistance and stress corrosion cracking resistance. Still furthermore, Si contributes to the improvement of the machinability and mechanical strength such as tensile strength, proof stress, impact strength, fatigue strength or the like. The above actions induce synergy effect on the grain refinement of the casting. Still furthermore, if added with Mn and Si, Si forms a Mn - Si intermetallic compound so as to improve the wear resistance. Co suppresses the coarsening of the grains induced under high-temperature heating condition and improves the strength of the copper alloy. That is, the addiction of Co preferably suppresses the grain growth induced when the Sn containing alloy is heated at the high-temperature, and then the metallic composition can be held minutely, and the fatigue resistance of the copper alloy after the high-temperature heating is improved. Considering the above facts, the contents of Mn, Al, Si and Co need to be in the above ranges.

In order to secure sufficient corrosion resistance, wear resistance, strength or the like, the first to fourth copper alloys need to form the above alloy compositions and to satisfy the condition (5). That is, the first to fourth copper alloys need to form phase structures (metal structures), in which the total content of α and δ-phases (and/or γ-phase) is 90% or more (preferably 98% or more, and more preferably 99% or more) by area ratio. However, if the content of δ-phase (and/or γ-phase) is excessive, the selective corrosion of the phase is generated so as to impair the corrosion resistance. In addition, even though δ and γ-phases improve the wear resistance and erosion resistance, δ and γ-phases also impair the ductility. Therefore, in order to maintain the strength, wear resistance and ductility in balance without causing the deterioration of the corrosion resistance in the above phase structure, it is preferable that the content of δ and γ-phases be controlled to be in the range of 0 to 20% (more preferably 0 to 10%, and further preferably 0 to 5%) by area ratio. If the grains are refined by adding Zr and P together, not only are δ and γ-phases divided and refined, but δ and γ-phases can also be distributed uniformly in the matrix, thereby improving various mechanical features and wear resistance (slidability) considerably.

In order for the first to fourth copper alloys to form the above phase structure and to satisfy the condition (6), it is preferable that the content of Sn be adjusted in consideration of the relationship between 'Sn' and 'Cu and the other additives'. That is, in order to refine the grains more effectively, not only do the contents of Sn or the like satisfy the conditions (2) to (4), but it is also preferable that the contents of Sn or the like be determined to satisfy the conditions (1) and (7) and the maximum of f0 (= [Cu] - 0.5[Sn] - 3[P] - 0.5([As] + [Sb]) + [Mg] + [Mn] - 1.8[Al] - 3.5[Si]) or the minimum of f4 (= [Zn] + 3[Sn]) be set as described above in the relationship of the contents of Cu, a main element, or the like in order to secure more excellent strength, corrosion resistance (erosion · corrosion resistance) and wear resistance. Meanwhile, considering the elongation, corrosion resistance and castability influenced by δ and γ-phases, it is preferable to control and set the minimum of f0 or the maximum of f4 as described above. In order to secure the above features, the concentration of Sn varies with the concentration of Cu.

In the first to fourth copper alloys, the grains are refined by adding Zr and P. In addition, high-quality casting products can be obtained and casting products by continuous casting such as horizontal continuous casting, upward (upcast) or the like can be provided and used practically by satisfying the condition (6), that is, by making the mean grain size in the macrostructure 300 µm or less (preferably 200 µm or less, and more preferably 100 µm or less; in addition, 60 µm or less in the microstructure) at the melt-solidification. When the grains are not refined, a plurality of heat treatments is required to eliminate dendrite structure peculiar to casting products and the segregation of Sn, and to divide and granulate δ and γ-phases, and the surface state of casting products become bad since the grains are coarsened. However, when the grains are refined as described above, the segregations are small and short, therefore, heat treatment is not required, and the surface state is good. In addition, since δ and γ-phases exist in the grain boundary even when δ and γ-phases are precipitated, the length of δ and γ-phases become short as the grains become finer and are distributed uniformly, therefore, specific treatment is not required to divide δ and γ-phases, or heat treatment is carried out to the minimum, if necessary. As described above, the number of processes required to manufacture a casting product is reduced considerably, therefore, the manufacturing cost can be decreased as much as possible. Meanwhile, if the condition (6) is satisfied, the following problems are not caused, therefore, the copper alloy exhibit excellent features. That is, when the size of each δ phase containing a large amount of Sn, a low-melting point metal, varies, and δ phase is distributed unevenly, the difference of the strength of δ-phase and α-phase in the matrix causes crack easily, and the ductility at the room temperature is impaired.

In addition, since the cold workability and hot workability are improved as δ and γ-phases are distributed uniformly and the length of δ and γ-phase or grain size becomes smaller, the first to fourth copper alloys can be used preferably for usages requiring caulking process (for example, there can be a case where caulking process is required when a hose nipple is installed).

Meanwhile, even though it is expected that the strength, corrosion resistance or the like is improved considerably as the content of Sn increases, for example, a large amount of Sn, exceeding 5 or 8 mass%, is added, on the other hand, Sn is segregated considerably, and thus crack, shrinkage cavity, blowhole or micro-porosity is highly likely to occur at the melt-solidification. However, when the grains are refined at the melt-solidification, such problems do not occur, and the further improvement of the strength, fatigue strength, seawater resistance, erosion · corrosion resistance or the like, achieved by adding a large amount of Sn, can be sought. In addition, Zr and P are added together only to refine the grains, not to impair the inherent features of the copper alloy. Furthermore, the identical or better features of the copper alloy having the same composition except that no Zr and P are contained as the grain-refining element are secured by the grain refinement achieved by the co-addition of Zr and P. In order to make the mean grain size small at the melt-solidification, it is required to determine the content of Zr or the like to satisfy the conditions (2) to (5), and it is preferable that the contents of Sn or the like be determined to satisfy the conditions (1) to (4) and (7) for the first to fourth copper alloys.

In addition, for the first to fourth copper alloys, there can be cases where scrap material is used as raw material, and when such scrap material is used, it is inevitable and permitted practically to contain impurities. However, when scrap material is a nickel plating material or the like, if Fe and/or Ni are contained as inevitable impurities, the content of Fe and/or Ni need to be controlled. That is, it is because Zr and P, which are useful for the grain refinement, are consumed by Fe and/or Ni, if the content of the impurities are large, thereby impeding the grain refinement. As a result, when either Fe or Ni is contained, the content of the impurity is controlled to be 0.25 mass% or less (preferably 0.1 mass% or less, and most preferably 0.06 mass% or less). Furthermore, when both Fe and Ni are contained, the content of Fe and Ni is controlled to be 0.3 mass% or less (more preferably 0.13 mass% or less, and most preferably 0.08 mass% or less).

In the preferred embodiments, the first to fourth copper alloys are provided in the form of, for example, casting product obtained by a casting process or plastic-worked product, which is the casting product that is further plastic-worked one or more times.

The casting product is provided in the form of, for example, wire, rod or hollow bar cast by horizontal continuous casting method, upward method or upcast method; or is provided as near net shaped product. In addition, the casting product is also provided in the form of casting, semi-solid metal casting, semi-solid metal molding, molten alloy forging or die casting molding. In this case, it is preferable that the molten alloy satisfy the conditions (12) and (13). If the solid phase is granulated in the semi-solid metal state, the semi-solid metal castability of the molten alloy becomes excellent, therefore, it is possible to carry out the semi-solid metal casting satisfactorily. Furthermore, even though the flowability of the molten liquid containing the solid phase at the final stage of solidification depends mainly on the shape of the solid phase, the viscosity of the liquid phase or the composition of the liquid phase, the moldability by casting (whether a robust casting can be cast even when high-precision or complex-shaped casting is required) is influenced considerably by the former factor (the shape of the solid phase). That is, if the solid phase begins to form dendrite network at the semi-solid metal state, the molten alloy containing the solid phase is hard to fill every corner of the mold, therefore, the moldability by casting deteriorates, and high-precision or complex-shaped casting products are difficult to obtain. Meanwhile, as the solid phase is granulated in the semi-solid metal state, and the shape of the solid phase becomes substantially spherical (circular in two dimension), the castability including the semi-solid metal castability is improved as much as the grain size becomes smaller, and thus a robust and high-precision or complex-shaped casting product can be obtained (it is needless to say that a high-quality semi-solid metal casting product can be obtained). Therefore, the semi-solid metal castability can be evaluated by checking the shape of the solid phase at the semi-solid metal state, and the other castabilities (complex-shaped castability, precision castability and melt-solidification forgeability) can be verified by the degree of the semi-solid metal castability. In general, when the dendrite network is divided in the crystal structure and the two-dimension shape of the solid phase is circular, substantially circular, oval, cross-like or polygonal at the semi-solid metal state, in which the solid phase occupies 30 to 80%, the semi-solid metal castability is good. In addition, particularly when either the mean grain size of the solid phase is 150 µm or less (preferably 100 µm or less, and more preferably 50 µm or less) or the mean maximum length of the solid phase is 200 µm or less (preferably 150 µm or less, and more preferably 100 µm or less) at the semi-solid metal state, in which the solid phase occupies 60%, the semi-solid metal castability is excellent.

In addition, a plastic-worked product is provided in the form of hot worked product (hot drawing worked product, hot forging worked product or hot rolling worked product) or cold worked product (cold rolling worked product, cold drawing worked product), and, in any case, proper heat treatment, cutting process or the like is performed additionally according to the necessity.

Specifically, the first fourth copper alloys are provided in the form of the following casting product or plastic-worked product, which is the casting product plastic-worked one or more times. For example, the casting product includes gear, worm gear, bearing, bush, impeller, common mechanical parts, metal fitting for water contact, joint or the like. In addition, the plastic-worked product includes electronic · electric device spring, switch, lead frame, connector, bellows, fuse grip, bush, relay, gear, cam, joint, flange, bearing, machine screw, bolt, nut, metal eyelet, heat exchanger tube sheet, heat exchanger, metallic mesh, sea net, breeding net, fish net, header material, washer, seawater condenser tube, ship parts shaft, seawater intake of a ship, metal fitting for water contact or the like.

Furthermore, in manufacturing the first to fourth copper alloys, the invention proposes a method of casting a copper alloy casting product having excellent strength, wear resistance and corrosion resistance, in which Zr is not added in the form of oxide and/or sulfide during casting by adding Zr (contained to further refine the grains and to make the grain refinement more stable) in the form of a copper alloy containing Zr right before pouring or at the final stage of raw material melting. It is preferable that the copper alloy containing Zr further contain one or more elements selected from P, Mg, Al, Sn, Mn and B as additives to a base material, that is, Cu - Zr alloy, Cu - Zn - Zr alloy or the alloy of Cu - Zr alloy and Cu - Zn - Zr alloy.

That is, in the casting process of the first to fourth copper alloys or the component (materials to be plastic-worked), the loss of Zr during the addition of Zr is reduced as much as possible by adding Zr in the form of granular, thin plate-like, rod-like or wire-like master alloy (copper alloy) right before pouring, thereby preventing Zr from being added in the form of oxide and/or sulfide during casting, which causes the lack of Zr required to refine the grains. In addition, when Zr is added right before pouring as described above, since the melting point of Zr is 800 to 1000°C higher than that of the copper alloy, it is preferable that Zr be used in the form of a low-melting point alloy, which is a granular (grain diameter: about 2 to 50 mm), thin plate-like (thickness: about 1 to 10 mm), rod (diameter: about 2 to 50 mm) or wire-like master alloy having the melting point close to that of the copper alloy and containing a large amount of necessary elements (for example, Cu - Zr alloy or Cu - Zn - Zr alloy containing 0.5 to 65 mass% of Zr; or an alloy further containing one or more elements selected from P, Mg, Al, Sn, Mn and B (the content of each element: 0.1 to 5 mass%) as additives to the base material, that is, the alloy of Cu - Zr alloy and Cu - Zn - Zr alloy) Particularly, in order to facilitate the melting by lowering the melting point and to prevent the loss of Zr due to the oxidation of Zr, it is preferable to use Zr in the form of an alloy that adopts Cu - Zn - Zr alloy containing 0.5 to 35 mass% of Zr and 15 to 50 mass% of Zn (preferably Cu - Zn - Zr alloy containing 1 to 15 mass% of Zr and 25 to 45 mass% of Zn) as the base. While depending on the proportion of Zr to P, which is added together with Zr, Zr impairs the electric · thermal conductive properties, that is, the inherent properties of the copper alloy. However, if the amount of Zr, not in the form of oxide and sulfide, is 0.039 mass% or less, particularly 0.019 mass% or less, the electric · thermal conductivity is seldom impaired by the addition of Zr, and further, the electric · thermal conductivity is less impaired as compared with when Zr is not added, if impaired.

In addition, in order to obtain the first to fourth copper alloys satisfying the condition (6), it is desirable to adjust properly the casing conditions, particularly, pouring temperature and cooling rate. That is, it is preferable that the pouring temperature be determined in the range of 20 to 250°C above the liquidus line temperature of the copper alloy (more preferably 25 to 150°C above the liquidus line temperature). That is, it is preferable that (the liquidus line temperature + 20°C) ≤ the pouring temperature ≤ (the liquidus line temperature + 250°C) and more preferable that (the liquidus line temperature + 25°C) ≤ the pouring temperature ≤ (the liquidus line temperature + 150°C). Generally, the pouring temperature is 1200°C or less, preferably 1150°C or less, and more preferably 1100°C or less. Even though the minimum pouring temperature is not limited as long as the molten alloy can fill every corner of the molder, as the pouring temperature is lowered, the grains are refined further. Meanwhile, it has to be understood that the above temperature conditions vary with the chemical composition of the alloy.

Since the grains are refined during the melt-solidification, the Sn-containing copper alloy of the invention can endure shrinkage during solidification, thereby inducing less casting crack. In addition, since air causing holes or porosity in the process of solidification can be removed easily to the outside, a robust casting product with no casting defect (since casting defect such as porosity or the like does not exist, dendrite network is not formed, the surface is smooth, and the shrinkage cavity is as thin as possible) can be obtained. Therefore, the invention can provide a casting product extremely rich in practicality or a plastic-worked product, which is the casting product plastic-worked one or more times.

In addition, since the crystals crystallized in the process of solidification are not dendrite, a typical shape of a casting structure, but broken arm-shaped, preferably circular, oval, polygonal or cross-like, the flowability of the molten alloy is improved, and thus the molten alloy can fill every corner of a molder having thin wall and complex shape.

Since the copper alloy of the invention considerably improves the strength, wear resistance (slidability), corrosion resistance, castability by refining the grains and distributing phases other than α-phase (δ and γ-phases generated by Sn) uniformly, the copper alloy can be used practically for a casting product demanding the above features (for example, gear, worm gear, bearing, bush, impeller, common mechanical parts, metal fitting for water contact, joint or the like) or a plastic-worked product that requires cold · hot process (for example, electric · electronic device spring, switch, lead frame, connector, bellow, fuse grip, bush, relay, gear, cam, joint, flange, bearing, machine screw, bolt, nut, metal eyelet, heat exchanger pipe, heat exchanger, metallic mesh, sea net, breeding net, fish net, header material, washer, seawater condenser tube, ship parts shaft, seawater intake of a ship, metal fitting for water contact or the like)

Even though the Cu - Sn alloy containing 8 mass% of Sn is used for a high class connector (for example, the connector of a cellular phone or the like) and thus it is highly demanded to strengthen the alloy by increasing the content of Sn, the strengthening causes a problem in the castability and thus cannot be realized. However, since the copper alloy of the invention can improve the castability of the alloy by refining the grains, the copper alloy can realize the above demand sufficiently and can be provided as a high-class connector. The Cu - Zn - Sn alloy containing Zn can do the same thing. Therefore, the invention can provide the Cu - Sn alloy or Cu - Zn - Sn alloy that satisfies inconsistent demands, that is, the improvement of strength and castability, thereby widening the usage of the Sn-containing copper alloy.

Furthermore, since the method of the invention can refine the grains by adding Zr and P together without inducing the problem caused by adding Zr in the form of oxide and sulfide, the copper alloy can be cast efficiently and preferably.

### Brief Description of the Drawings

Fig. 1 includes photographs of etched surface (cross-section) of a copper alloy for embodiment No. 5, in which Fig. 1 (A) shows a macrostructure, and Fig. 1(B) shows a microstructure.
Fig. 2 includes photographs of etched surface (cross-section) of a copper alloy for comparative example No. 107, in which Fig. 2(A) shows a macrostructure, and Fig. 2(B) shows a microstructure.
Fig. 3 includes photographs of etched surface (cross-section) of a copper alloy for embodiment No. 18, in which Fig. 3(A) shows a macrostructure, and Fig. 3(B) shows a microstructure.
Fig. 4 includes photographs of etched surface (cross-section) of a copper alloy for comparative example No. 112, in which Fig. 4(A) shows a macrostructure, and Fig. 4(B) shows a microstructure.
Fig. 5 includes X-ray micro-analyzer photographs of etched surface (cross-section) of a copper alloy for embodiment No. 18, in which Fig. 5(A) shows a composition image, and Fig. 5(B) shows a distribution of Sn.
Fig. 6 includes X-ray micro-analyzer photographs of etched surface (cross-section) of a copper alloy for comparative example No. 112, in which Fig. 6(A) shows a composition image, and Fig. 6(B) shows a distribution of Sn.
Fig. 7 includes cross-sectional views showing a result of Tatur test, in which Fig. 7(A) shows 'good'-ranked copper alloy, Fig. 7(B) shows 'fair'-ranked copper alloy, and Fig. 7(C) shows 'bad'-ranked copper alloy.

### Best Mode for Carrying Out the invention

As embodiments, copper alloy materials having the compositions listed in Table 1 are melted in an electric furnace, and then the molten alloy is poured into an iron-made molder preheated up to 200°C so as to manufacture cylindrical (diameter: 40 mm, length: 280 mm) ingots (hereinafter referred to as 'copper alloys for embodiment') Nos. 1 to 33. When the ingots for the reference copper alloys , which do not contain Zn, are manufactured, Zr is added to the molten alloy in the form of granular Cu - Zr alloy (cubic body with several mm long sides) right before pouring. As a result, Zr is prevented from being added in the form of oxide and sulfide. Likewise, when the first to fourth copper alloys are manufactured, Zr is added to the molten alloy in the form of granular Cu - Zn - Zr alloy (cubic body with several mm long sides) right before pouring. Furthermore, the pouring temperatures are set to be 100°C above the liquidus line temperatures of the copper alloys.

In addition, as comparative examples, copper alloy materials having the compositions listed in Table 2 are melted in an electric furnace, and then the molten alloy is poured into an iron-made molder preheated up to 200°C under the same conditions as those of the embodiments so as to cast cylindrical (diameter: 40 mm, length: 280 mm) ingots (hereinafter referred to as 'copper alloys for comparative example') Nos. 101 to 116.

No. 10 Specimen defined in JIS Z 2201 is taken from the copper alloys for embodiment and the copper alloys for comparative example, and then tensile strength test is performed on the specimens by AMSLER universal testing machine so as to measure the tensile strength (N/mm²), 0.2% proof stress (N/mm²) and elongation (%). The result is illustrated in Tables 3 and 4.

In order to check the corrosion resistance of the copper alloys for embodiment and the copper alloys for comparative examples, the following erosion · corrosion test and dezincification corrosion test defined in 'ISO 6509' are performed.

That is, for the erosion · corrosion test, 3% saline solution (30°C) is sprayed to the specimens taken from the copper alloys for embodiment and the copper alloys for comparative example through a 1.9 mm diameter nozzle at a speed of 11 m/sec in a direction perpendicular to the axis of the species for 96 hours, and then the mass loss (mg/cm²) is measured. The mass loss is the difference of mass per one square centimeter (mg/ cm²) between the mass of the original specimen and the mass of the specimen sprayed with the 3% saline solution for 96 hours. The result of the erosion · corrosion test is illustrated in Tables 3 and 4.

For the dezincification corrosion test of 'ISO 6509', the specimens taken from the copper alloys for embodiment and the copper alloys for comparative example are molded with phenol resin so as to make the exposed surfaces of the specimens face perpendicular to the extension direction, and then the surfaces are polished with Emery paper up to No. 1200. After that, the surfaces are ultrasonic-cleaned in pure water and then dried. The obtained corrosion test specimens are soaked in an aqueous solution of 1.0% Cupric Chloride Dihydrate (CuCl2 · 2H₂O) for 24 hours at the temperature of 75°C. After that, the specimens are removed from the aqueous solution, and then the maximum depth of dezincification corrosion, that is, the maximum dezincification corrosion depth (µm), is measured. The result is illustrated in Tables 3 and 4.

From the above test results, it is verified that the embodiments are more excellent in both of the mechanical properties (strength, elongation or the like) and the corrosion resistance than the comparative examples. In addition, even though it is possible to consider that the elongation is reduced by the grain refinement, the result of the tensile test shows that the elongation of the Sn-containing copper alloy of the invention is not reduced, inversely, improved.

In addition, in order to evaluate the cold workability of the copper alloys for embodiment and the copper alloys for comparative example, the following cold compression test is performed.

That is, cylindrical specimens with a diameter of 5 mm and a height of 7.5 mm are cut by a lathe from the copper alloys for embodiment and the copper alloys for comparative example, and then compressed by AMSLER universal test machine so as to evaluate the cold workability on the basis of the relationship between the existence of crack and the compression rate (processing rate). The result is illustrated in Tables 3 and 4. In the tables, specimens having crack at the compression rate of 50% are denoted by 'x' as a meaning of bad cold workability; specimens having no crack at the compression rate of 65% are denoted by 'O' as a meaning of good cold workability; and specimens having no crack at the compression rate of 50%, however, having crack at the compression rate of 65% are denoted by 'Δ' as a meaning of fair cold workability.

Furthermore, in order to evaluate the hot workability of the copper alloys for embodiment and the copper alloys for comparative example, the following high-temperature compression test is performed.

That is, cylindrical specimens with a diameter of 15 mm a height of 25 mm are taken from the copper alloys for embodiment and the copper alloys for comparative example by a lathe and then held at the temperature of 700°C for 30 minutes. After that, the specimens are hot-compressed while the processing rate is varied so as to evaluate the hot workability on the basis of the relationship between the processing rate and the crack. The result is illustrated in Tables 3 and 4. In the tables, specimens having no crack at the processing rate of 65% are denoted by 'O' as a meaning of good hot workability; specimens having a small number of little cracks at the processing rate of 65%, however, having no crack at the processing rate of 55% are denoted by 'Δ' as a meaning of fair hot workability; and specimens having crack at the processing rate of 55% are denoted by 'x' as a meaning of bad hot workability.

From the above test results, it is verified that the copper alloys for embodiment are excellent in both of the cold workability and the hot workability.

Still furthermore, for the copper alloys for embodiment and the copper alloys for comparative example, the phase structure at the room temperature after melt-solidification is checked, and the area ratio (%) of γ and δ-phases is measured by image analysis. That is, the structures of the copper alloys, 200 times magnified by an optical microscope, are expressed in the binary system with an image processing software 'WinROOF' so as to measure the area ratio of each phase. The area ratio is measured in three dimensional, and the average value of the area ratio is regarded as the phase ratio of each phase. The result is illustrated in Table 1 and 2. All copper alloys for embodiment satisfy the condition (5). In addition, Tables 3 and 4 illustrate the primary crystals, crystallized during melt-solidification in casting process, for the copper alloys for embodiment and the copper alloys for comparative example. Since having α-phase primary crystal, all copper alloys for embodiment satisfy the condition (8).

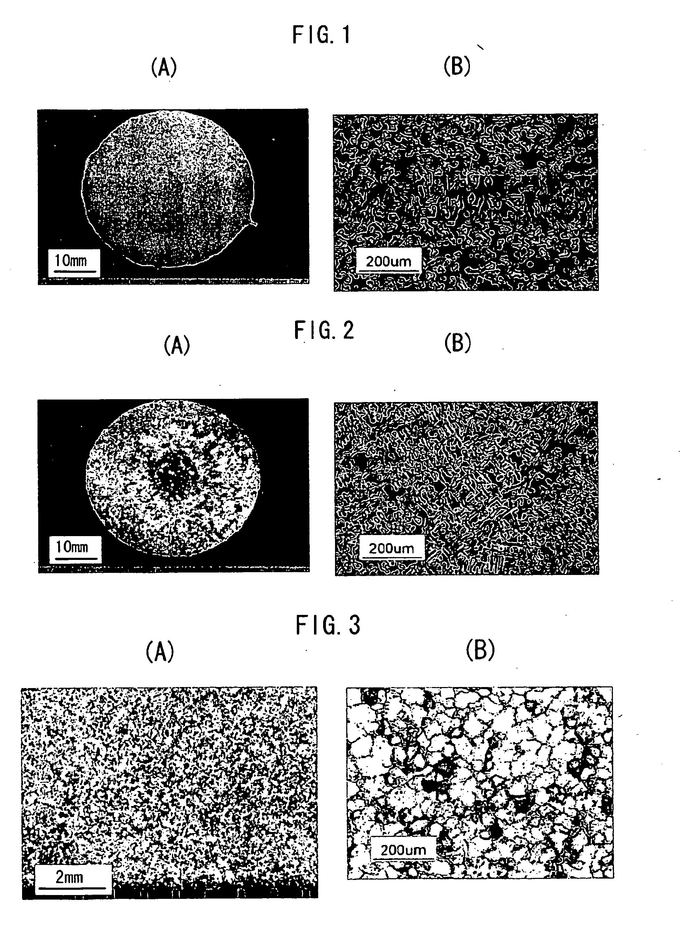

Still furthermore, for the copper alloys for embodiment and the copper alloys for comparative example, the mean grain size (µm) during melt-solidification is measured. That is, a casting product is divided, and the cross-section is etched by nitric acid. After that, the mean grain size of the microstructure displayed on the etched surface is measured. The measurement is based on the comparative method for estimating average grain size of wrought copper and copper alloys of JIS H0501, herein, grains having the diameter of more than 0.5 mm are observed by eyes, and grains having the diameter of 0.5 mm or less is 7.5 times magnified for observation after the divided surface is etched by nitric acid. In addition, grains having the diameter of less than about 0.1 mm are etched by a mixed liquid of hydrogen peroxide and ammonia water and then 75 times magnified by an optical microscope for observation. The result is illustrated in Tables 3 and 4, and all copper alloys for embodiment satisfy the condition (6). Furthermore, it is verified that the copper alloys for embodiment satisfy the conditions (10) to (11). Figs. 1 to 6 show an example.

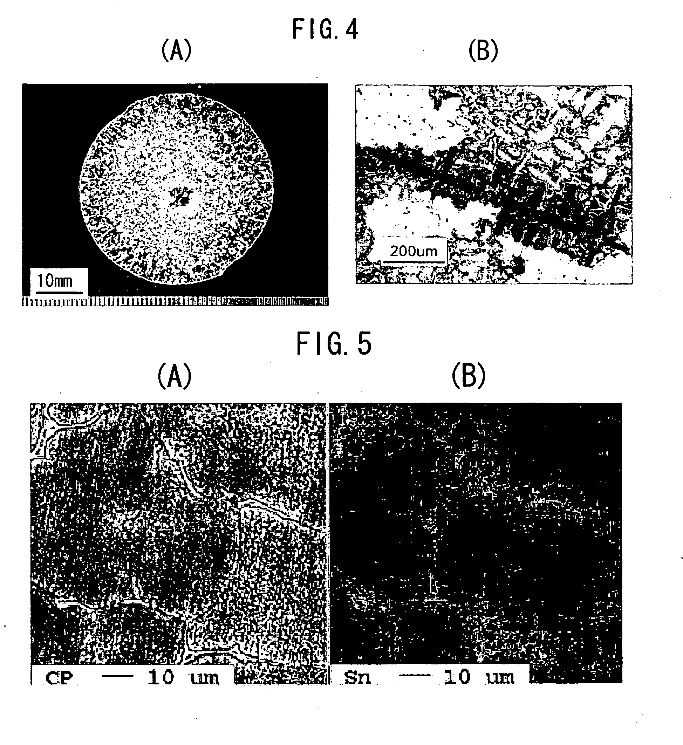

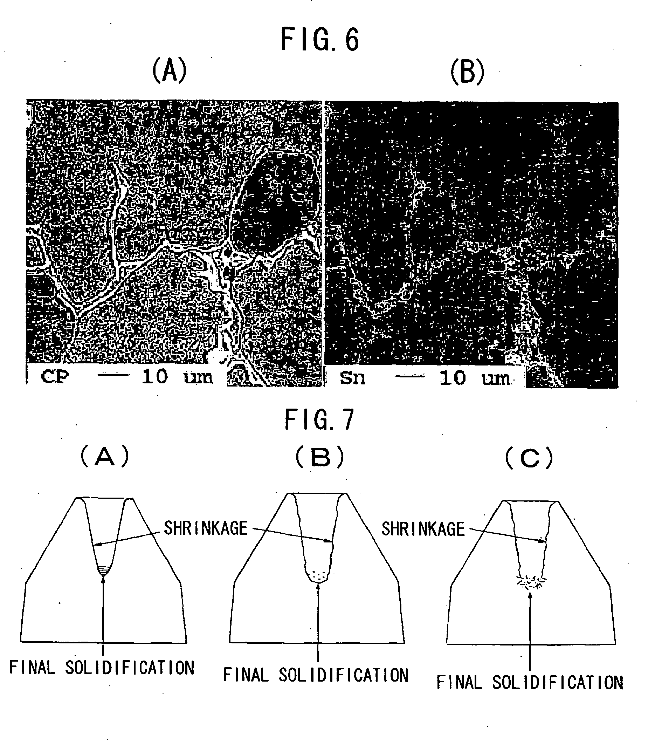

Fig. 1 includes a photograph of the macrostructure (Fig. 1(A)) and a photograph of the microstructure (Fig. 1(B)) of the copper alloy for embodiment No. 5 For reference only and outside the scope of the invention that contains no Zn. Fig. 2 includes a photograph of the macrostructure (Fig. 2(A)) and a photograph of the microstructure (Fig. 2(B)) of the copper alloy for comparative example No. 107, Cu - Sn alloy containing no Zn, like the copper alloy No. 5, and the same content of Sn as that of the copper alloy No. 5. Fig. 3 includes a photograph of the macrostructure (Fig. 3(A)) and a photograph of the microstructure (Fig. 3(B) of the copper alloy for embodiment No. 18 that contains Zn. Fig. 4 includes a photograph of the macrostructure (Fig. 4(A)) and a photograph of the microstructure (Fig. 4(B)) of the copper alloy for comparative example No. 112, Cu - Zn - Sn alloy that contains Zn and the same content of Sn as that of the copper alloy No. 18. From Figs. 1 to 4, it is evident that the copper alloys for embodiment Nos. 5 For reference only and outside the scope of the invention and 18 satisfy the conditions (10) and (11); however, the copper alloys for comparative example Nos. 107 and 112 do not satisfy the conditions (10) and (11). Furthermore, Fig. 5 includes X-ray micro-analyzer photographs of the casting products of Embodiment No. 18, in which Fig. 5(A) shows the composition and Fig. 5(B) shows the distribution of Sn. Still furthermore, Fig. 6 includes X-ray micro-analyzer photographs of the casting products of Comparative Example No. 112, in which Fig. 6(A) shows the composition and Fig. 6(B) shows the distribution of Sn. From Fig. 5, it is evident that, since the high Sn-concentration parts (white parts in Fig. 5(B)) of similar small sizes are distributed uniformly, the casting product of Embodiment No. 18 satisfies the condition (11). On the other hand, in the copper alloy for Comparative example No. 112, as shown in Fig. 6, the high Sn-concentration parts (white parts in Fig. 6(B)) have irregular sizes and are distributed non-uniformly, thereby not satisfying the condition (11).

From the above facts, it can be understood that the grains can be refined effectively and the high Sn-concentration part can be refined and distributed by adding a proper amount of Zr and P under the above conditions. In addition, comparing the results of the corrosion resistance test or the like and the result of the Tatur test, described below, it is evident that the copper alloys for Embodiment Nos. 5 For reference only and outside the scope of the invention and 18 have better corrosion resistance, castability or the like than the copper alloys for comparative example Nos. 107 and 112. Therefore, it can be understood that satisfying the conditions (10) and (11) is very important in improving the corrosion resistance, castability or the like.

Still furthermore, even though it is considered that the castability is improved by satisfying the condition (6), that is, by refining the grains, in order to verify this fact, the following Tatur shrinkage test and semi-solid metal castability test are performed.

That is, Tatur shrinkage test is performed on the molten alloys (the molten alloys of the copper alloy materials having the compositions listed in Table 1 or 2) used in casting the copper alloys for embodiment and the copper alloys for comparative example, and then the castability is evaluated by checking the shape of internal shrunk portion and the existence of casting defect such as porosity, hole, shrinkage cavity or the like in the vicinity of the internal shrunk portion. The castability is evaluated to be 'good' for copper alloys having a smooth internal shrunk portion and no defect such as porosity or the like in the finally solidified portion as shown in Fig. 7(A), 'bad' for copper alloys having a non-smooth, that is, remarkably uneven, internal shrunk portion and defect such as porosity or the like in the finally solidified portion as shown in Fig. 7(C), and 'fair' for copper alloys evaluated neither 'good' nor 'bad' as shown in Fig. 7(B). The result is illustrated in Tables 5 to 8. In the tables, 'good' is denoted by 'O', 'fair' is denoted by 'Δ', and 'bad' is denoted by '×'. Meanwhile, it is also verified that the grain size of the macrostructure of the casting products obtained for the Tatur test substantially corresponds to the measuring result of the grain size of the copper alloys for embodiment and the copper alloys for comparative examples as described above.

In the semi-solid metal castability test, raw material used in casting the copper alloys for embodiment and the copper alloys for comparative example is charged into a crucible; heated up to a temperature, at which the raw material forms a semi-solid metal (solid phase ratio: about 60%); held at the temperature for 5 minutes; and then cooled rapidly (water cooling). After that, the shape of the solid phase at the semi-solid metal state is investigated so as to evaluate the semi-solid metal castability. The result is illustrated in Tables 3 and 4, and it is verified that the copper alloys for embodiment have excellent semi-solid metal castability. Meanwhile, in the tables, copper alloys having the mean grain size of the solid phase of 150 µm or less or the mean maximum grain length of 300 µm or less are denoted by 'O' as a meaning of good semi-solid metal castability; copper alloys having the grain size of the solid phase not satisfying the above condition, but, having no dendrite network therein are denoted by 'Δ' as a meaning of industrially satisfactory semi-solid metal castability; and copper alloys having dendrite network therein are denoted by 'x' as a meaning of bad semi-solid metal castability.

It can be understood from the above test results that the copper alloys for embodiment have better castability and semi-solid metal castability than the copper alloys for comparative example.

Meanwhile, a new casting product (hereinafter referred to as 'recycled casting product') is cast by using the copper alloy casting product (including the specimen used in the tensile strength test) No. 18 (hereinafter referred to as 'product casting') obtained in the embodiment as raw material. That is, the product casting (the copper alloy casting No. 18) is re-melted at the temperature of 1030°C in a state that the molten alloy is coated with charcoal and held for 5 minutes, and then Cu - Zn - Zr alloy containing 3 mass% of Zr is further added to replenish the oxidation loss of Zr in melting, which is expected to be 0.0015 mass%. After that, the product casting is poured into a molder. As a result, the obtained recycled casting product has the content of Zr, almost equal to that of the product casting No. 18, raw material, (0.0096 mass%) and the mean grain size, almost equal to that of the product casting No. 18, that is, 35 µm. From the above fact, it is verified that the copper alloy casting product of the invention can effectively use surplus part or unnecessary part such as runner or the like generated in casting as recycling material without impairing the grain refinement. Therefore, the surplus part or unnecessary part such as molten alloy passage can be used as replenishing material, which is charged during continuous operation, and perform the continuous operation effectively and economically.

**[Table 1]**

| | Copper alloy No. | Alloy composition (mass%) | | | | | | | | | | Area ratio (%) |
|---|---|---|---|---|---|---|---|---|---|---|---|---|
| | | Cu | Zn | Zr | P | Sn | f0 | f1 | F2 | f3 | f4 | α+γ+δ |
| E m b o d i m e n t | 1* | 95.7 | | 0.033 | 0.05 | 4.2 | 93.5 | 1.5 | 382 | 252 | 12.6 | 100 |
| | 2* | 93.8 | | 0.028 | 0.07 | 6.1 | 90.5 | 2.5 | 654 | 261 | 18.3 | 100 |
| | 3* | 92 | | 0.023 | 0.06 | 7.9 | 87.9 | 2.6 | 1030 | 395 | 23.7 | 100 |
| | 4* | 89.8 | | 0.0084 | 0.06 | 10.1 | 84.6 | 7.1 | 3607 | 505 | 30.3 | 100 |
| | 5* | 89.8 | | 0.014 | 0.06 | 10.1 | 84.6 | 4.3 | 2164 | 505 | 30.3 | 100 |
| | 6* | 89.8 | | 0.022 | 0.06 | 10.1 | 84.6 | 2.7 | 1377 | 505 | 30.3 | 100 |
| | 7* | 89.7 | | 0.028 | 0.07 | 10.2 | 84.4 | 2.5 | 1093 | 437 | 30.6 | 100 |
| | 8* | 89.8 | | 0.035 | 0.06 | 10.1 | 84.6 | 1.7 | 866 | 505 | 30.3 | 100 |
| | 9* | 88.1 | | 0.02 | 0.06 | 11.8 | 82.0 | 3.0 | 1770 | 590 | 35.4 | 100 |
| | 10* | 84.7 | | 0.019 | 0.12 | 15.1 | 76.8 | 6.3 | 2384 | 378 | 45.3 | 100 |
| | 11* | 89.7 | | 0.019 | 0.07 | 10.1 | 84.5 | 3.7 | 1595 | 433 | 30.3 | 100 |
| | 12* | 89.7 | | 0.018 | 0.06 | 10.1 | 84.4 | 3.3 | 1683 | 505 | 30.3 | 100 |
| | 13* | 89.8 | | 0.021 | 0.07 | 10.1 | 84.5 | 3.3 | 1443 | 433 | 30.3 | 100 |
| | 14 | 78 | 21.60 | 0.019 | 0.06 | 0.32 | 77.7 | 3.2 | 1187 | 376 | 22.6 | 100 |
| | 15 | 75.5 | 24.38 | 0.018 | 0.05 | 0.05 | 75.3 | 2.8 | 1363 | 491 | 24.5 | 100 |
| | 16 | 72.8 | 25.95 | 0.0029 | 0.05 | 1.2 | 72.1 | 17.2 | 10189 | 591 | 29.5 | 100 |
| | 17 | 73.2 | 25.55 | 0.0068 | 0.04 | 1.2 | 72.5 | 5.9 | 4287 | 729 | 29.2 | 100 |
| | 18 | 73.4 | 25.34 | 0.0095 | 0.05 | 1.2 | 72.7 | 5.3 | 3046 | 579 | 28.9 | 100 |
| | 19 | 73.1 | 25.62 | 0.016 | 0.06 | 1.2 | 72.3 | 3.8 | 1827 | 487 | 29.2 | 100 |
| | 20 | 73.4 | 25.32 | 0.035 | 0.05 | 1.2 | 72.7 | 1.4 | 826 | 578 | 28.9 | 100 |
| | 21 | 73 | 25.76 | 0.021 | 0.015 | 1.2 | 72.4 | 0.7 | 1398 | 1958 | 29.4 | 100 |
| | 22 | 72.9 | 25.73 | 0.018 | 0.15 | 1.2 | 71.9 | 8.3 | 1630 | 196 | 29.3 | 100 |
| | 23 | 87.2 | 9.33 | 0.018 | 0.05 | 3.4 | 85.4 | 2.8 | 1085 | 391 | 19.5 | 100 |
| | 24 | 85 | 12.14 | 0.018 | 0.04 | 2.8 | 83.5 | 2.2 | 1141 | 514 | 20.5 | 100 |
| | 25 | 88.3 | 8.61 | 0.017 | 0.05 | 3 | 86.7 | 2.9 | 1036 | 352 | 17.6 | 100 |
| | 26 | 64.1 | 31.78 | 0.018 | 0.05 | 0.05 | 63.4 | 2.8 | 1774 | 639 | 31.9 | 100 |
| | 27 | 77.2 | 20.73 | 0.018 | 0.05 | 0.1 | 73.6 | 2.8 | 1168 | 421 | 21.0 | 100 |
| | 28 | 73 | 25.71 | 0.022 | 0.026 | 1.2 | 72.3 | 1.2 | 1332 | 1127 | 29.3 | 100 |
| | 29 | 70.2 | 29.63 | 0.018 | 0.05 | 0.05 | 70.0 | 2.8 | 1655 | 596 | 29.8 | 100 |
| | 30 | 65.1 | 33.94 | 0.019 | 0.05 | 0.8 | 64.5 | 2.6 | 1913 | 727 | 36.3 | 100 |
| | 31 | 77.8 | 20.09 | 0.018 | 0.05 | 0.5 | 72.1 | 2.8 | 1200 | 432 | 21.6 | 100 |
| | 32 | 78 | 19.80 | 0.015 | 0.06 | 2.1 | 76.8 | 4.0 | 1740 | 435 | 26.1 | 100 |
| | 33 | 74.3 | 23.68 | 0.018 | 0.06 | 1.4 | 72.5 | 3.3 | 1549 | 465 | 27.9 | 100 |

| | | | | | | | | | | | | |
|---|---|---|---|---|---|---|---|---|---|---|---|---|
| * For reference only and outside the scope of the invention | | | | | | | | | | | | |

**[Table 2]**

| | Copper alloy No. | Alloy composition (mass%) | | | | | | | | | | Area ratio (%) |
|---|---|---|---|---|---|---|---|---|---|---|---|---|
| | | Cu | Zn | Zr | P | Sn | f0 | f1 | F2 | f3 | f4 | α+γ+δ |
| C o m p a r a t i v e | 101 | 93.8 | | 0.0003 | 0.06 | 6.1 | 90.6 | 200.0 | 61000 | 305 | 18.3 | 100 |
| | 102 | 93.6 | | 0.0013 | 0.21 | 6.1 | 89.9 | 161.5 | 14077 | 87 | 18.3 | 100 |
| | 103 | 92 | | 0.035 | | 7.9 | 88.1 | 0 | 677 | | 23.7 | 100 |
| | 104 | 91.9 | | 0.0004 | 0.05 | 8 | 87.8 | 125.0 | 60000 | 480 | 24.0 | 100 |
| | 105 | 91.8 | 0.0015 | 0.23 | 7.9 | 87.2 | 153.3 | 15800 | 103 | 23.7 | 100 | |
| | 106 | 89.8 | 0.0008 | 0.06 | 10.1 | 84.6 | 75.0 | 37875 | 505 | 30.3 | 100 | |
| | 107 | 89.8 | | 0.004 | | 10.1 | 84.8 | 0 | 758 | | 30.3 | 100 |
| | 108 | 89.8 | | 0.019 | 0.008 | 10.1 | 84.7 | 0.4 | 1595 | 3788 | 30.3 | 100 |
| | 109 | 89.6 | | 0.07 | 0.06 | 10.2 | 84.3 | 0.9 | 437 | 510 | 30.6 | 100 |
| | 110 | 89.8 | | 0.038 | 0.014 | 10.2 | 84.7 | 0.4 | 805 | 2186 | 30.6 | 100 |
| | 111 | 73 | 25.74 | | 0.06 | 1.2 | 72.2 | | | 489 | 29.3 | 100 |
| | 112 | 73.1 | 25.62 | 0.0007 | 0.08 | 1.2 | 72.3 | 114.3 | 41742 | 365 | 29.2 | 100 |
| | 113 | 72.8 | 25.97 | 0.023 | 0.008 | 1.2 | 72.2 | 0.3 | 1286 | 3696 | 29.6 | 100 |
| | 114 | 73 | 25.77 | 0.035 | | 1.2 | 72.4 | 0 | 839 | | 29.4 | 100 |
| | 115 | 88 | 8.55 | 0.0008 | 0.05 | 3.4 | 86.2 | 62.5 | 23437 | 375 | 18.7 | 100 |
| | 116 | 60 | 38.72 | 0.018 | 0.06 | 1.2 | 59.2 | 3.3 | 2351 | 705 | 42.3 | 84 |

**[Table 3]**

| | COPPER ALLOY No. | MAXIMUM CRISTAL | MEAN GRAIN SIZE (µm) | TATUR TEST | MAXIMUM CORROSION DEPTH (µm) | NOT -BILITY | COLD PROCESS IBILITY | COROSION-EROSION TEST MASS LOSS (mg/cm²) | TENSILE STRENGTH (N/mm²) | PROOF STRESS (N/mm²) | ELON GATION (%) | SEMI-SOLID METAL CASTABIL ITY |
|---|---|---|---|---|---|---|---|---|---|---|---|---|
| | 1* | α | 150 | ○ | | ○ | ○ | | | | | |
| | 2* | α | 70 | ○ | | ○ | ○ | | 346 | 136 | 42 | Δ |
| | 3* | α | 50 | ○ | | ○ | ○ | | 366 | 144 | 44 | |
| | 4* | α | 55 | ○ | | Δ | ○ | 21 | 362 | 139 | 38 | |
| | 5* | α | 20 | ○ | | ○ | ○ | 20 | 377 | 164 | 42 | ○ |
| | 6* | α | 25 | ○ | | ○ | ○ | | 388 | 172 | 43 | |
| | 7* | α | 35 | ○ | | Δ | ○ | | 381 | 166 | 43 | |
| | 8* | α | 75 | ○ | | Δ | ○ | | 363 | 142 | 39 | |
| | 9* | α | 15 | ○ | | | ○ | | | | | |
| | 10* | α | 20 | ○ | | | Δ | | | | | |
| | 11* | α | 20 | ○ | | ○ | ○ | | | | | |
| | 12* | α | 25 | ○ | | Δ | ○ | | | | | |
| | 13* | α | 25 | ○ | | ○ | ○ | | | | | |
| | 14 | α | 90 | ○ | 10 OR LESS | ○ | ○ | | | | | |
| | 15 | α | 80 | ○ | 30 | ○ | ○ | | | | | |
| | 16 | | α 150 | ○ | 60 | Δ | ○ | | 323 | 105 | 28 | |
| | 17 | α | 75 | ○ | 10 OR LESS | ○ | ○ | | 324 | 115 | 36 | |
| E M B O D I M E N T | 18 | α | 35 | ○ | 10 OR LESS | ○ | ○ | 21 | 349 | 138 | 38 | ○ |
| | ₁₉ 9 | ₐ | 3₀ | 0 | 10 OR LESS | ○ | ○ | 21 | 354 | 142 | 38 | ○ |
| | 20 | α | 80 | ○ | 20 | ○ | ○ | | 326 | 113 | 35 | |
| | 21 | α | 200 | ○ | 70 | Δ | ○ | | 311 | 106 | 28 | |
| | 22 | α | 120 | ○ | 40 | ○ | Δ | | 302 | 112 | 17 | |
| | 23 | α | 90 | ○ | | Δ | ○ | | | | | |
| | 24 | α | 75 | ○ | | ○ | ○ | | | | | |
| | 25 | α | 80 | ○ | | ○ | ○ | | | | | |
| | 26 | α | 45 | ○ | | ○ | ○ | | 475 | 174 | 30 | |
| | 27 | α | 35 | ○ | | ○ | ○ | 22 | 375 | 138 | 34 | ○ |
| | 28 | α | 120 | ○ | 30 | Δ | ○ | | 318 | 110 | 34 | |
| | 29 | α | 50 | ○ | 20 | ○ | ○ | | | | | |
| | 30 | α | 45 | ○ | 20 | ○ | ○ | | | | | |
| | 31 | α | 55 | ○ | 10 | ○ | ○ | 21 | 398 | 136 | 33 | |
| | 32 | α | 50 | ○ | | ○ | ○ | | | | | |
| | 33 | α | 35 | ○ | 20 | ○ | ○ | | | | | |

| | | | | | | | | | | | | |
|---|---|---|---|---|---|---|---|---|---|---|---|---|
| * For reference only and outside the scope of the invention | | | | | | | | | | | | |

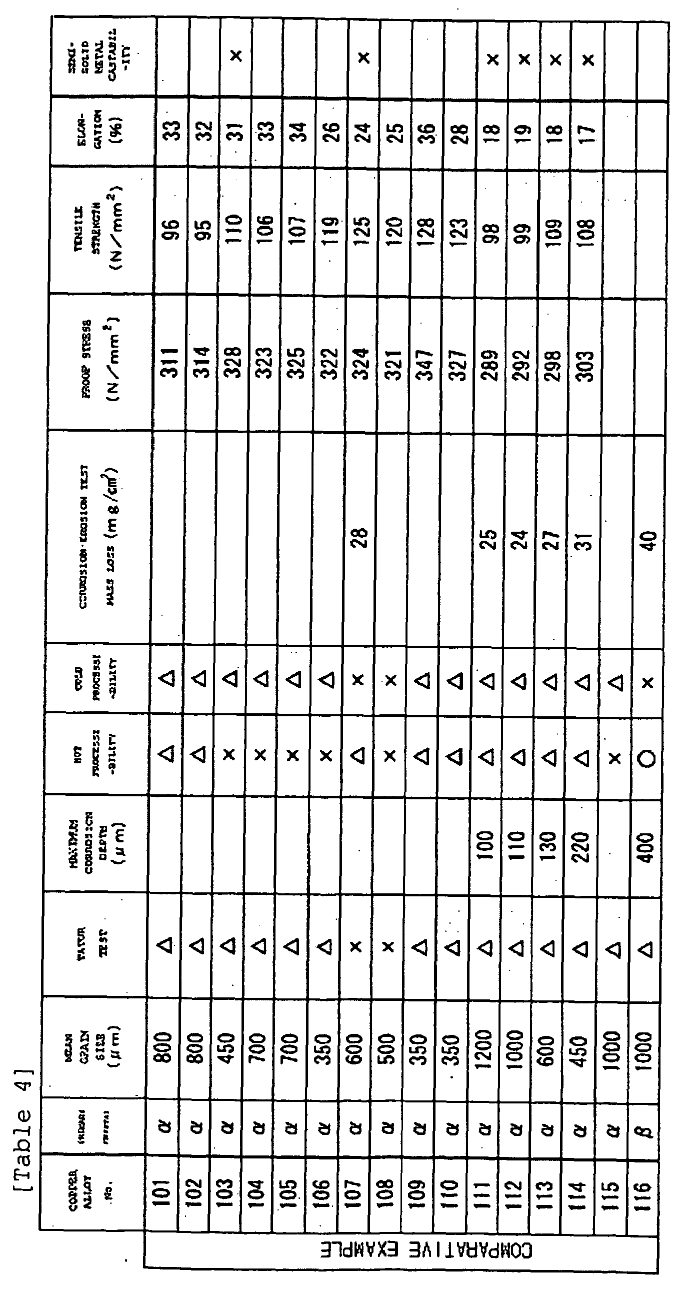

### Industrial Applicability

The copper alloy according to the invention is used as, specifically, the following casting product, plastic-worked product, that is, plastic-worked casting product, or structural material thereof. For example, the casting product includes gear, worm gear, bearing, bush, impeller, common mechanical parts, metal fitting for water contact, joint or the like or the structural materials thereof, and the plastic-worked product includes electronic · electric device spring, switch, lead frame, connector, bellow, fuse grip, bush, relay, gear, cam, joint, flange, bearing, machine screw, bolt, nut, hatome, heat exchanger tube sheet, heat exchanger, metallic mesh, sea, net, breeding net, fish net, header material, washer, seawater condenser tube, ship parts shaft, seawater intake of a ship or metal fitting for water contact or like or the structural material thereof.

## Claims

1. A Sn-containing copper alloy, comprising:
Sn: 0.01 to 16 mass%, Zr: 0.001 to 0.049 mass%, P: 0.01 to 0.25 mass%, Zn: 0.01 to 38 mass%; optionally one or more elements selected from Mn: 0.05 to 4 mass%, Al: 0.01 to 3 mass%, Si: 0.01 to 1.9 mass%, Co: 0.005 to 0.1 mass%, and optionally one or more elements selected from As : 0.02 to 0.2 mass%, Sb: 0.02 to 0.2 mass% and Mg: 0.001 to 0.2 mass%; optionally Fe and/or Ni as inevitable impurities, wherein when either of Fe or Ni is included, the content thereof is limited to be 0.25 mass% or less, and when both of Fe and Ni are included, the total content thereof is limited to be 0.3 mass% or less; and Cu: remainder
wherein
f0 = [Cu] - 0.5[Sn] - 3 [P] - 0.5([As] + [Sb]) + [Mg] + [Mn] - 1.8[Al] - 3.5[Si] = 61 to 97,
f1 = [P] / [Zr] = 0.5 to 100,
f2 = ([Zn] + 3[Sn]) / [Zr] = 30 to 15000,
f3 = ([Zn] + 3[Sn]) / [P] = 3 to 2500
f4 = [Zn] + 3[Sn] = 10 to 48
(a content of element 'a' is represented as [a] mass%) are satisfied,
α-phase and one or both of γ-phase and δ-phase are included and the total content of the α-phase and one or both of γ-phase and δ-phase reaches 90% or more by area ratio, and
a mean grain size in a macrostructure after melt-solidification is 300 µm or less.

2. The Sn-containing copper alloy according to claim 1,
wherein a primary crystal is α-phase during the melt-solidification.

3. The Sn-containing copper alloy according to claim 1 or 2,
wherein peritectic reaction is generated during the melt-solidification.

4. The Sn-containing copper alloy according to claim 1,
wherein dendrite network is divided in crystal structure and a two-dimensional shape of grain is circular, substantially circular, oval, cross-like, acicular or polygonal during the melt-solidification.

5. The Sn-containing copper alloy according to claim 1,
wherein the α-phase in a matrix is minutely divided, and the δ and γ-phases or high Sn-concentration parts generated by segregation are distributed uniformly in the matrix.

6. The Sn-containing copper alloy according to claim 1,
wherein, in a semi-solid metal state including 30 to 80% of solid phase, at least the dendrite network is divided in the crystal structure and the two-dimensional shape of the solid phase is circular, substantially circular, oval, cross-like, acicular or polygonal.

7. The Sn-containing copper alloy according to claim 6,
wherein, in the semi-solid metal state including 60% of the solid phase, a mean grain size of the solid phase is 150 µm or less and/or a mean maximum grain length of the solid phase is 200 µm or less.

8. The Sn-containing copper alloy according to any one of claims 1 to 7,
wherein the copper alloy is a casting product (including a semi-solid metal casting product) obtained in a casting process or a plastic-worked product, which is the casting product plastic-worked additionally one or more times.

9. The Sn-containing copper alloy according to claim 8,
wherein the casting product is a wire, rod or hollow bar cast by horizontal continuous casting method, upward method or upcast method.

10. The Sn-containing copper alloy according to claim 8,
wherein the casting product is a casting product, semi-solid metal casting product, semi-solid metal molding product, molten alloy forging product or die casing molding product.

11. The Sn-containing copper alloy according to claim 8,
wherein the plastic-worked product is a hot extruding product, hot forging product or hot rolling product.

12. The Sn-containing copper alloy according to claim 8,
wherein the plastic-worked product is a cold rolling product or cold drawing product.

13. The Sn-containing copper alloy according to claim 8,
wherein the casting product is a gear, a worm gear, a bearing, a bush, an impeller, common mechanical parts, a metal fitting for water contact or a joint.

14. The Sn-containing copper alloy according to claim 8, wherein the plastic-worked product is electronic electric device spring, switch, lead frame, connector, bellow, fuse grip, bush, relay, gear, cam, joint, flange, bearing, machine screw, bolt, nut, metal eyelet, heat exchanger pipe, heat exchanger, metallic mesh, sea net, breeding net, fish net, header material, washer, seawater condenser tube, ship parts shaft, seawater intake of a ship or metal fitting for water contact.

15. A method of manufacturing the Sn-containing copper alloy according to any one of claims 1 to 14,
wherein, in a casting process, Zr is added in a form of copper alloy containing Zr in order to prevent Zr from being added in a form of oxide and/or sulfide.

16. The method of manufacturing the Sn-containing copper alloy according to claim 15,
wherein the copper alloy containing Zr is one of Cu - Zr alloy, Cu - Zn - Zr alloy, Cu - Zr alloy, to which one or more element selected from P, Mg, Al, B, Sn and Mn are added, and Cu - Zn - Zr alloy, to which one or more element selected from P, Mg, Al, B, Sn and Mn are added.

## Patentansprüche

1. Sn-haltige Kupferlegierung, umfassend
Sn: 0,01 bis 16 Massen-%, Zr: 0,001 bis 0,049 Massen-%, P: 0,01 bis 0,25 Massen-%, Zn: 0,01 bis 38 Massen-%; wahlweise ein oder mehrere Elemente, ausgewählt aus Mn: 0,05 bis 4 Massen-%, Al: 0,01 bis 3 Massen-%, Si: 0,01 bis 1,9 Massen-%, Co: 0,005 bis 0,1 Massen-% und wahlweise ein oder mehrere Elemente, ausgewählt aus As: 0,02 bis 0,2 Massen-%, Sb: 0,02 bis 0,2 Massen-% und Mg: 0,001 bis 0,2 Massen-%; wahlweise Fe und/oder Ni als unvermeidbare Verunreinigungen, worin dann, wenn eines von Fe oder Ni enthalten ist, der Gehalt davon auf 0,25 Massen-% oder weniger begrenzt ist und wenn beide von Fe und Ni enthalten sind, der gesamte Gehalt davon auf 0,3 Massen-% oder weniger begrenzt ist; und Cu: Rest,
worin
f0 - [Cu] - 0,5[Sn] - 3[P] - 0,5([As] + [Sb]) + [Mg] + [Mn] - 1,8[Al] - 3,5[Si] = 61 to 97,
f1 = [P] / [Zr] = 0,5 to 100,
f2 = ([Zn] + 3[Sn]) / [Zr] = 30 to 15000,
f3 = ([Zn] + 3[Sn]) / [P] = 3 to 2500
f4 = [Zn] + 3[Sn] = 10 to 48
(ein Gehalt des Elementes "a" wird als [a] Massen-% dargestellt) erfüllt sind,
eine α-Phase und eine oder beide von einer γ-Phase und δ-Phase enthalten sind und der Gesamtgehalt der α-Phase und eine oder beide von einer γ-Phase und δ-Phase 90% oder mehr bezogen auf das Flächenverhältnis erreichen, und
eine mittlere Korngröße in einer Makrostruktur nach Schmelzverfestigung 300 µm oder weniger ist.

2. Sn-haltige Kupferlegierung nach Anspruch 1, worin ein Primärkristall eine α-Phase während der Schmelzverfestigung ist.

3. Sn-haltige Kupferlegierung nach Anspruch 1 oder 2, worin eine peritektische Reaktion während der Schmelzverfestigung erzeugt wird.

4. Sn-haltige Kupferlegierung nach Anspruch 1, worin ein Dendritnetzwerk in Kristallstruktur unterteilt ist und eine zweidimensionale Form eines Korns kreisförmig, im Wesentlichen kreisförmig, oval, kreuzförmig, nadelförmig oder polygonal während der Schmelzverfestigung ist.

5. Sn-haltige Kupferlegierung nach Anspruch 1, worin die α-Phase in einer Matrix fein zerteilt ist und die δ- und γ-Phasen oder Teile mit hoher Sn-Konzentration, erzeugt durch Segregation, einheitlich in der Matrix verteilt sind.

6. Sn-haltige Kupferlegierung nach Anspruch 1, worin in einem halbfesten Metallzustand, umfassend 30 bis 80% der festen Phase, zumindest das Dendritnetzwerk in die Kristallstruktur unterteilt ist und die zweidimensionale Form der festen Phase kreisförmig, im Wesentlichen kreisförmig, oval, kreuzartig, nadelförmig oder polygonal ist.

7. Sn-haltige Kupferlegierung nach Anspruch 6, worin in dem halbfesten Metallzustand, umfassend 60% der festen Phase, eine mittlere Korngröße der festen Phase 150 µm oder weniger ist und/oder eine mittlere maximale Kornlänge der festen Phase 200 µm oder weniger ist.

8. Sn-haltige Kupferlegierung nach einem der Ansprüche 1 bis 7, worin die Kupferlegierung ein Gussprodukt (einschließlich einem halbfesten Metallgussprodukt), erhalten in einem Gussverfahren oder ein kunststoffverarbeitetes Produkt ist, dass das Gussprodukt ist, das ein oder mehrere Male zusätzlich kunststoffverarbeitet ist.

9. Sn-haltige Kupferlegierung nach Anspruch 8, worin das Gussprodukt ein Draht, Stab oder hohle Stange ist, gegossen durch ein horizontales kontinuierliches Gussverfahren, Aufwärtsverfahren oder Aufgießverfahren.

10. Sn-haltige Kupferlegierung nach Anspruch 8, worin das Gussprodukt ein Gussprodukt, halbfestes Metallgussprodukt, halbfestes Metallformprodukt, geschmolzenes Metall-Schmiedeprodukt oder Düsenguss-Formprodukt ist.

11. Sn-haltige Kupferlegierung nach Anspruch 8, worin das kunststoffverarbeitete Produkt ein Heißextrusionsprodukt, Heißschmiedeprodukt oder Heißwalzenprodukt ist.

12. Sn-haltige Kupferlegierung nach Anspruch 8, worin das kunststoffverarbeitete Produkt ein kaltgewalztes Produkt oder kaltgezogenes Produkt ist.

13. Sn-haltige Kupferlegierung nach Anspruch 8, worin das Gussprodukt ein Antrieb, Schneckenantrieb, Auflage, Buchse, Antriebsrad, allgemeines mechanisches Teil, Metallformstück für Wasserkontakt oder eine Verbindung ist.

14. Sn-haltige Kupferlegierung nach Anspruch 8, worin das kunststoffverarbeitete Produkt eine Feder für eine elektronische-elektrische Vorrichtung, Schalter, Leitungsrahmen, Anschluss, Blasebalg, Sicherungsgriff, Buchse, Relais, Antrieb, Nocke, Verbindung, Flansch, Auflage, Maschinenschraube, Bolzen, Mutter, Metallöse, Wärmeaustauschrohr, Wärmetauscher, Metallgitter, Meeresnetz, Brutnetz, Fischernetz, Verteilermaterial, Wäscher, Meereswasser-Kondensatorrohr, Schiffsteileschaft, Meereswasseraufnahme für ein Schiff oder Beschläge für den Wasserkontakt ist.

15. Verfahren zur Herstellung der Sn-haltigen Kupferlegierung nach einem der Ansprüche 1 bis 14, worin in einem Gussverfahren Zr in einer Form einer Kupferlegierung, umfassend Zr, zugegeben wird, um zu verhindern, dass Zr in einer Form von Oxid und/oder Sulfid zugegeben wird.

16. Verfahren zur Herstellung der Sn-haltigen Kupferlegierung nach Anspruch 15, worin die Kupferlegierung, umfassend Zr, eine von Cu-Zr-Legierung, Cu-Zn-Zr-Legierung, Cu-Zr-Legierung ist, zu der ein oder mehrere Elemente, ausgewählt aus P, Mg, Al, B, Sn und Mn zugegeben sind, und Cu-Zn-Zr-Legierung ist, zu der ein oder mehrere Elemente, ausgewählt aus P, Mg, Al, B, Sn und Mn zugegeben ist.

## Revendications

1. Alliage de cuivre contenant du Sn, comprenant :
Sn : 0,01 à 16% en masse, Zr : 0,001 à 0,049% en masse, P : 0,01 à 0,25% en masse, Zn : 0,01 à 38% en masse ; éventuellement un ou plusieurs éléments choisis parmi Mn : 0,05 à 4% en masse, Al : 0,01 à 3% en masse, Si : 0,01 à 1,9% en masse, Co : 0,005 à 0,1% en masse, et éventuellement un ou plusieurs éléments choisis parmi As : 0,02 à 0,2% en masse, Sb : 0,02 à 0,2% en masse et Mg : 0,001 à 0,2% en masse; éventuellement Fe et/ou Ni comme impuretés inévitables, dans lequel lorsque l'un de Fe ou Ni est inclus, la teneur de celui-ci sera limitée à 0,25% en masse ou moins, et lorsque Fe et Ni à la fois sont inclus, la teneur totale de ceux-ci est limitée à 0,3% masse ou moins ; et Cu : reste
dans lequel
f0 = [Cu] - 0,5[Sn] - 3[P] - 0,5([As] + [Sb]) + [Mg] + [Mn] - 1,8[Al] - 3,5[Si] = 61 à 97,
f1 = [P] / [Zr] = 0,5 à 100,
f2 = ([Zn] + 3[Sn]) / [Zr] = 30 à 15 000,
f3 = ([Zn] + 3[Sn]) / [P] = 3 à 2 500
f4 = [Zn] + 3[Sn] = 10 à 48
(une teneur de l'élément « a » est représenté par [a]% en masse) sont satisfaites,
une phase α et une ou les deux phase γ et phase δ sont incluses et la teneur totale de la phase α et de l'une ou des deux phase γ et phase δ atteint 90% ou plus en rapport de surfaces, et
une taille moyenne des grains dans une macrostructure, après fusion-solidification est de 300 µm ou moins.

2. Alliage de cuivre contenant du Sn selon la revendication 1,
dans lequel un cristal primaire est une phase α pendant la fusion-solidification.

3. Alliage de cuivre contenant du Sn selon la revendication 1 ou 2,
dans lequel une réaction péritectique est générée pendant la fusion-solidification.

4. Alliage de cuivre contenant du Sn selon la revendication 1,
dans lequel un réseau de dendrites est divisé dans la structure cristalline et une forme bidimensionnelle de grain est circulaire, sensiblement circulaire, ovale, en forme de croix, aciculaire ou polygonale pendant la fusion-solidification.

5. Alliage de cuivre contenant du Sn selon la revendication 1,
dans lequel la phase α dans une matrice est minutieusement divisée, et les phases δ et γ ou les parties à concentration élevée en Sn générées par ségrégation sont réparties uniformément dans la matrice.

6. Alliage de cuivre contenant du Sn selon la revendication 1,
dans lequel, dans un état de métal semi-solide incluant 30 à 80% de phase solide, le réseau de dendrites au moins est divisé dans la structure cristalline et la forme bidimensionnelle de la phase solide est circulaire, sensiblement circulaire, ovale, en forme de croix, acirculaire ou polygonale.

7. Alliage de cuivre contenant du Sn selon la revendication 6,
dans lequel, dans l'état de métal semi-solide incluant 60% de la phase solide, une taille moyenne des grains de la phase solide est de 150 µm ou moins et/ou une longueur maximale moyenne des grains de la phase solide est de 200 µm ou moins.

8. Alliage de cuivre contenant du Sn selon l'une quelconque des revendications 1 à 7,
dans lequel l'alliage de cuivre est un produit coulé (y compris un produit coulé de métal semi-solide) obtenu dans un procédé de coulée ou un produit écroui, qui est le produit coulé, écroui en outre une ou plusieurs fois.

9. Alliage de cuivre contenant du Sn selon la revendication 8,
dans lequel le produit coulé est un fil, une tige ou une barre creuse coulé par un procédé de coulée continue horizontale, un procédé ascendant ou un procédé de coulée verticale.

10. Alliage de cuivre contenant du Sn selon la revendication 8,
dans lequel le produit coulé est un produit coulé, un produit coulé de métal semi-solide, un produit moulé de métal semi-solide, un produit forgé d'alliage fondu ou un produit moulé sous pression.

11. Alliage de cuivre contenant du Sn selon la revendication 8,
dans lequel le produit écroui est un produit d'extrusion à chaud, un produit forgé à chaud ou un produit laminé à chaud.

12. Alliage de cuivre contenant du Sn selon la revendication 8,
dans lequel le produit écroui est un produit laminé à froid ou un produit d'étirage à froid.

13. Alliage de cuivre contenant du Sn selon la revendication 8,
dans lequel le produit coulé est un engrenage, un engrenage à vis sans fin, un roulement, une douille, une turbine, des pièces mécaniques courantes, un raccord en métal pour contact avec l'eau ou un joint.

14. Alliage de cuivre contenant du Sn selon la revendication 8, dans lequel le produit écroui est un ressort de dispositif électrique électronique, un commutateur, un cadre de plomb, un connecteur, un soufflet, un serrage de fusible, une douille, un relais, un engrenage, une came, un joint, une bride, un roulement, une vis de machine, un boulon, un écrou, un oeillet métallique, un tube d'échangeur thermique, un échangeur thermique, un treillis métallique, un filet de mer, un filet d'élevage, un filet de pêche, un matériel de collecteur, une rondelle, un tube de condenseur d'eau de mer, l'arbre de parties d'un navire, une prise d'eau de mer d'un navire ou un raccord en métal pour contact avec l'eau.

15. Procédé de fabrication de l'alliage de cuivre contenant du Sn selon l'une quelconque des revendications 1 à 14,
dans lequel, dans un procédé de coulée, du Zr est ajouté sous une forme d'alliage de cuivre contenant du Zr, afin d'empêcher que le Zr soit ajouté sous une forme d'oxyde et/ou de sulfure.

16. Procédé de fabrication de l'alliage de cuivre contenant du Sn selon la revendication 15,
dans lequel l'alliage de cuivre contenant du Zr est l'un parmi un alliage de Cu - Zr, un alliage de Cu - Zn - Zr, un alliage de Cu - Zr, auquel un ou plusieurs éléments choisis parmi P, Mg, Al, B, Sn et Mn sont ajoutés, et un alliage de Cu - Zn - Zr, auquel un ou plusieurs éléments choisis parmi P, Mg, Al, B, Sn et Mn sont ajoutés.
